# EUROPÄISCHE PATENTANMELDUNG

(11) **EP 1 336 420 A2**
(43) Veröffentlichungstag der Anmeldung: **20.08.2003**
(21) Anmeldenummer: 03003078.7
(22) Anmeldetag: 12.02.2003
(51) Int. Cl.: A61M 16/04

(54) **Mundstück für medizinische Zwecke**

(30) Priorität: 13.02.2002 DE 20202227 U
(71) Anmelder: Kolb, Dieter Dr. med., 66133 Saarbrücken (DE)
(72) Erfinder: Kolb, Dieter Dr. med., 66133 Saarbrücken (DE)
(74) Vertreter: Wieske, Thilo

(57) **Zusammenfassung**

Die vorliegende Erfindung betrifft ein Mundstück (1) für medizinische Zwecke, insbesondere zur Erleichterung beim Einführen und Herausnehmen des Intubationsschlauches.

Dazu weist das Mundstück (1) zumindest ein Aufbissstück (2,3) auf, das beim Gebrauch des Mundstücks (1) im Bereich der Kiefer an der Position der Backenzähne angeordnet ist zwischen dem Ober- und Unterkiefer zumindest auf einer Seite der Kiefer, wobei durch das Aufbissstück (2,3) der Ober- und Unterkiefer auseinander gehalten werden.

## Beschreibung

Die vorliegende Erfindung betrifft ein Mundstück für medizinische Zwecke nach dem Oberbegriff des Anspruchs 1.

Derartige medizinische Zwecke können beispielsweise die Anästhesie, die Bronchoskopie, die Gastroskopie oder auch Maßnahmen der Ersten Hilfe sein. Ebenso können die medizinischen Zwecke auch in einer Behandlung zu suchen sein, die mittels eines Intubationsschlauches durchgeführt wird.

Derzeit kommt es bei Narkosen beim Einführen und Herausnehmen des Intubationsschlauches in einer bestimmten Zahl von Fällen zu einem Abbrechen oder dem Verlust von Zähnen. Dies liegt darin begründet, dass mit einem gewissen Kraftaufwand Unter- und Oberkiefer des Patienten auseinander gedrückt werden müssen, um den Intubationsschlauch in die Luftröhre einführen bzw. wieder entfernen zu können. Dadurch kann es zu Traumen der Zähne, des Mundvorhofes und der Kiefergelenke, zum Teil auch mit Auswirkungen auf die Halswirbelsäule, kommen.

Der vorliegenden Erfindung liegt die Aufgabe zu Grunde, die beschriebenen Probleme zu vermeiden.

Diese Aufgabe wird nach der vorliegenden Erfindung gemäß Anspruch 1 gelöst, indem das Mundstück zumindest ein Aufbissstück aufweist, das beim Gebrauch des Mundstücks im Bereich der Kiefer an der Position der Backenzähne angeordnet ist zwischen dem Ober- und dem Unterkiefer zumindest auf einer Seite der Kiefer, wobei durch das Aufbissstück der Ober- und der Unterkiefer auseinander gehalten werden.

Dabei weist das Mundstück zumindest im Bereich der Vorderzähne eine Öffnung auf, durch die ein Intubationsschlauch oder auch ein Endoskop geführt werden kann.

Es erweist sich bei diesem Mundstück als vorteilhaft, dass auch bei relaxiertem Patienten die optimale Mundöffnung sicher gestellt werden kann. Die beschriebenen Läsionen beim Einführen und Herausnehmen des Intubationsschlauches können vorteilhaft vermieden werden, weil durch das Mundstück Unter- und Oberkiefer so weit auseinander gehalten werden, dass der Intubationsschlauch bzw. das Endoskop ohne Probleme ein- und ausgeführt werden können.

Bei der Ausgestaltung nach Anspruch 2 sind die Backenzähne die großen Backenzähne (Molaren).

Dabei erweist es sich als vorteilhaft, dass durch das Aufbissstück einerseits Unter- und Oberkiefer sicher auseinander gehalten werden können. Andererseits wird durch die Position des Aufbissstücks im Bereich der Molaren die Öffnung des Mundes ermöglicht, so dass ein Intubationsschlauch oder auch ein Endoskop einfach eingebracht werden kann.

Bei der Ausgestaltung nach Anspruch 3 sind zwei Aufbissstücke vorhanden, von denen jeweils eines auf je einer Seite der Kiefer angeordnet ist, wobei die beiden Aufbissstücke miteinander verbunden sind.

Dadurch kann das Auseinanderhalten von Unter- und Oberkiefer verbessert werden. Ein Mundstück, bei dem das Aufbissstück lediglich auf einer Seite der Kiefer wirkt, kann beispielsweise bei Operationen am Kiefer vorteilhaft verwendet werden, weil der Kieferbereich durch das Mundstück dann nur teilweise verdeckt ist. Indem bei der Ausgestaltung nach Anspruch 3 die beiden Aufbissstücke auf beiden Seiten der Kiefer angeordnet sind, kann weiterhin von vorne in einfacher Weise ein Intubationsschlauch oder auch ein Endoskop eingeführt werden.

Die Verbindung zwischen den beiden Aufbissstücken kann so realisiert sein, dass diese Verbindung entlang des Unterkiefers und/oder entlang des Oberkiefers verläuft. Die Verbindung muss dabei lediglich wenigstens eine hinreichend große Öffnung offen lassen, durch die ein Intubationsschlauch, ein Endoskop oder ähnliches durchgeführt werden kann.

Bei der Ausgestaltung nach Anspruch 4 besteht die Verbindung der beiden Aufbissstücke aus einem flexiblen Material, das die Zähne zumindest teilweise umgibt.

Dadurch kann vorteilhaft ein Schutz für die Zähne realisiert werden. Dies betrifft die Schneidezähne, die Eckzähne, die kleinen Backenzähne und die großen Backenzähne (Incisivi, Canini, Prämolaren und Molaren). Indem die Verbindung aus flexiblem Material besteht, kann das Mundstück einfach unterschiedlichen Gebissgrößen bzw. Gebissformen angepasst werden. Neben dem Schutz der Zähne ist auch ein Schutz des Mundvorhofes, der Mundhöhle, der Zunge, der Lippen, Wangen sowie des Zahnfleisches erreichbar.

Bei der Ausgestaltung nach Anspruch 5 verläuft die Verbindung zumindest teilweise hinter den Zähnen.

Entlang des Oberkiefers verläuft die Verbindung dann palatinal, entlang des Unterkiefers lingual. Dadurch kann vorteilhaft ein Herausdrücken des Mundstücks durch die Zunge bei dem relaxierten Patienten vermieden werden.

Bei der Ausgestaltung nach Anspruch 6 verläuft die Verbindung zumindest teilweise vor den Zähnen.

Dadurch werden vorteilhaft die Zähne effizient geschützt bei der Behandlung des Patienten und insbesondere auch beim Einführen bzw. Herausnehmen eines Intubationsschlauches, Endoskopes oder ähnlichem.

Bei der Ausgestaltung nach Anspruch 7 besteht die Verbindung aus zwei Verbindungselementen, wobei ein Verbindungselement entlang des Oberkiefers verläuft und ein weiteres Verbindungselement entlang des Unterkiefers.

Dadurch wird zum einen eine gute Stabilität des Mundstücks erreicht. Weiterhin wird ein besonders effektiver Schutz der Zähne erreicht, wenn die Verbindung die Zähne umgibt. Bei der Ausgestaltung nach Anspruch 7 sind dann sowohl die Zähne des Unterkiefers wie auch die Zähne des Oberkiefers geschützt.

Bei der Ausgestaltung nach Anspruch 8 weist das Mundstück im Außenbereich im Bereich der Vorderzähne ein Befestigungselement auf.

Dadurch kann vorteilhaft beispielsweise eine Trachealkanüle an dem Mundstück befestigt werden. Die Befestigung kann beispielsweise mittels eines Karabinerhakens erfolgen. Das Befestigungselement kann unter Umständen auch als Griff benutzt werden, um das Mundstück bei dem relaxierten Patienten zu entfernen. Dies kann noch durch einen leichten Druck auf die Kinnspitze unterstützt werden.

Das vorgeschlagene Mundstück eignet sich als Standard der Methodenbeschreibung für Narkosen und enorale Endoskopien - auch für eine Zertifizierung nach der ISO 9901.

Mit dem vorgeschlagenen Mundstück können Patienten bei Operationen auch eventuell vorhandene Zahnprothesen anbehalten, so dass über das Mundstück eine bessere Abstützung realisiert wird.

Das Mundstück kann beispielsweise in drei verschiedenen Standard-Größen hergestellt werden. Mit diesen drei Größen lassen sich alle üblichen Kieferverhältnisse von Patienten abdecken. Ggf. können auch noch Ausführungsformen hergestellt werden für Patienten mit totalen Prothesen oder Teilbezahnung.

Die Ausmessung des passenden Mundstücks kann beispielsweise erfolgen, indem mit einem Messinstrument die Breite der Kiefer in Höhe der ersten Molaren sowie die Länge der Kiefer von der Schneidekante der Frontzähne bis zu den ersten Molaren gemessen wird. Durch diese Werte lässt sich das passende Mundstück auswählen.

Nach der Auswahl kann das passende Mundstück bei noch nicht relaxiertem Patienten eingesetzt werden. Dabei sollte darauf geachtet werden, dass die Lippen nicht gequetscht sind. Die zumindest annähernd maximale Mundöffnung beim Einsetzen und später bei einer Narkose wird durch den Aufbiss im Molarenbereich beibehalten. Dadurch ist auch gewährleistet, dass das Mundstück in dieser optimalen Position verbleibt. Es kann dann die Narkoseeinleitung (mit eingesetztem Mundstück) erfolgen und danach sofort - ohne Manipulationen zur Öffnung des Mundes - intubiert oder endoskopiert werden.

Neben den genannten Vorteilen, ergibt sich durch das Mundstück auch ein Schutz der behandelnden Person vor eventuellen Bissverletzungen.

Durch die Investition in das Mundstück ergeben sich nachfolgend auch Kostenreduktionen. Diese betreffen zum einen Haftpflichtfälle für Krankenhäuser und Behandler wegen Schäden am Patienten, Reduzierung der Beiträge für die Berufsgenossenschaft wegen eines geringeren Verletzungsrisikos der Behandler, Unfallvermeidung und dadurch bedingten Arbeitsausfall sowie Kosten für Nach- und Mehrbehandlungen.

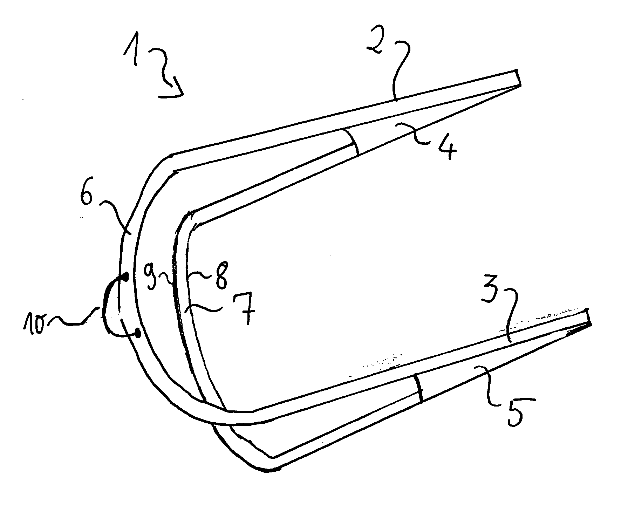

Ein Ausführungsbeispiel der Erfindung ist in der Zeichnung dargestellt. Die einzige Figur zeigt dabei ein Mundstück 1. Dieses Mundstück 1 weist dabei zwei Aufbissbereiche 2 und 3 auf, die bei eingesetztem Mundstück im Bereich der Position der Backenzähne im Kiefer liegen. Zwischen den Aufbissbereichen 2 und 3 befindet sind jeweils zwischen dem Aufbissbereich für den Oberkiefer und den Unterkiefer ein Distanzstück 4 und 5. Mit diesen Distanzstücken 4 und 5 werden der Oberkiefer und der Unterkiefer auseinander gehalten. Es ist selbstverständlich auch möglich, die Distanzstücke 4 und 5 jeweils mit den zugehörigen Aufbissbereichen 2 und 3 einstückig auszubilden.

Weiterhin sind Verbindungselemente 6 und 7 zu sehen, mit denen die Aufbissbereiche 2 und 3 sowohl entlang des Oberkiefers (mit dem Verbindungselement 6) wie auch entlang des Unterkiefers (mit dem Verbindungselement 7) miteinander verbunden sind.

Es ist zu sehen, dass die Aufbissbereiche 2 und 3 so angeordnet sind, dass im Gebrauchszustand des Mundstücks 1 jeweils einer dieser Aufbissbereiche 2, 3 auf jeweils einer Seite der Kiefer angeordnet ist. Dabei ergibt sich eine Position im Bereich der großen Backenzähne (Molaren).

Die Verbindungselemente 6 und 7 bestehend vorzugsweise aus einem elastischen Material wie beispielsweise Silikonkautschuk. Vorteilhaft sind die Distanzstücke 4 und 5 aus einem festeren Kunststoff gebildet, so dass Ober- und Unterkiefer sicher auseinander gehalten werden.

Die Verbindungselemente 6 und 7 legen sich aufgrund der Materialeigenschaften vorteilhaft um die Zähne herum, so dass diese durch die Verbindungselemente 6 und 7 geschützt sind. Im Zusammenhang mit dem Verbindungselement 7 ist dabei ein Bereich mit der Bezugsziffer 8 markiert, der hinter den Zähnen des Unterkiefers zum Anliegen kommt (lingual). Dadurch kann ein Herausdrücken des Mundstücks mit der Zunge verhindert werden. Der Bereich 9 legt sich vor den Zähnen an, so dass diese geschützt werden. Die im Zusammenhang mit dem Unterkiefer beschriebenen Verhältnisse ergeben sich ersichtlich beim Oberkiefer genau so.

Weiterhin ist noch zu sehen, dass an dem Verbindungselement 6 ein Befestigungselement 10 angebracht ist. An diesem Befestigungselement 10 kann eine Trachealkanüle mittels eines Karabinerhakens befestigt werden.

## Patentansprüche

1. Mundstück für medizinische Zwecke,
**dadurch gekennzeichnet, dass** das Mundstück (1) zumindest ein Aufbissstück (2, 3) aufweist, das beim Gebrauch des Mundstücks (1) im Bereich der Kiefer an der Position der Backenzähne angeordnet ist zwischen dem Ober- und dem Unterkiefer zumindest auf einer Seite der Kiefer, wobei durch das Aufbissstück (2, 3) der Ober- und der Unterkiefer auseinander gehalten werden.

2. Mundstück nach Anspruch 1,
**dadurch gekennzeichnet, dass** die Backenzähne die großen Backenzähne (Molaren) sind.

3. Mundstück nach Anspruch 1 oder 2,
**dadurch gekennzeichnet, dass** zwei Aufbissstücke (2, 3) vorhanden sind, von denen jeweils eines beim Gebrauch des Mundstücks auf je einer Seite der Kiefer angeordnet ist, wobei die beiden Aufbissstücke (2, 3) miteinander verbunden sind (6, 7).

4. Mundstück nach Anspruch 3,
**dadurch gekennzeichnet, dass** die Verbindung (6, 7) der beiden Aufbissstücke (2, 3) aus einem flexiblen Material besteht, das die Zähne zumindest teilweise umgibt.

5. Mundstück nach Anspruch 3 oder 4,
**dadurch gekennzeichnet, dass** die Verbindung (6, 7) zumindest teilweise hinter den Zähnen verläuft (8).

6. Mundstück nach einem der Ansprüche 3 oder 4,
**dadurch gekennzeichnet, dass** die Verbindung (6, 7) im Bereich des Unterkiefers zumindest teilweise vor den Zähnen verläuft (9).

7. Mundstück nach Anspruch 5 und 6,
**dadurch gekennzeichnet, dass** die Verbindung aus zwei Verbindungselementen (6, 7) besteht, wobei ein Verbindungselement (6) entlang des Oberkiefers verläuft und ein weiteres Verbindungselement (7) entlang des Unterkiefers.

8. Mundstück nach einem der Ansprüche 1 bis 7,
**dadurch gekennzeichnet, dass** das Mundstück (1) im Außenbereich im Bereich der Vorderzähne ein Befestigungselement (10) für eine Trachealkanüle aufweist.
